# FASCICULE DE BREVET EUROPEEN

(11) **EP 0 625 153 B1**
(45) Date de publication et mention de la délivrance du brevet: **06.09.1995**
(21) Numéro de dépôt: 93904153.9
(22) Date de dépôt: 04.02.1993
(51) Int. Cl.: C07D 471/04, A61K 31/44, B01D 15/08

(54) **DERIVE DE L'ISOINDOLINONE, SA PREPARATION ET LES COMPOSITIONS PHARMACEUTIQUES QUI LE CONTIENNENT**
ISOINDOLINON-DERIVAT, SEINE HERSTELLUNG UND DIESES ENTHALTENDE PHARMAZEUTISCHE ZUSAMMEN SETZUNGEN
ISOINDOLINONE DERIVATIVE, PREPARATION THEREOF, AND PHARMACEUTICAL COMPOSITIONS CONTAINING SAME

(30) Priorité: 07.02.1992 FR 9201382
(43) Date de publication de la demande: 23.11.1994
(73) Titulaire: RHONE-POULENC RORER S.A., 92160 Antony (FR)
(72) Inventeur: BARREAU, Michel, F-91230 Montgeron (FR); CHEVE, Michel, F-91450 Soisy-sur-Seine (FR); DUBROEUCQ, Marie-Christine, F-95880 Enghien-les-Bains (FR); DUTRUC-ROSSET, Gilles, F-75012 Paris (FR); MANFRE, Franco, F-94450 Limeil-Brevannes (FR)
(74) Mandataire: Pilard, Jacques
(86) Numéro de dépôt international: FR9300113
(87) Numéro de publication internationale: WO9316074

(56) Documents cités:
- EP-A- 0 174 858
- EP-A- 0 274 930

## Description

La présente invention concerne un nouveau dérivé de l'isoindolinone de formule : 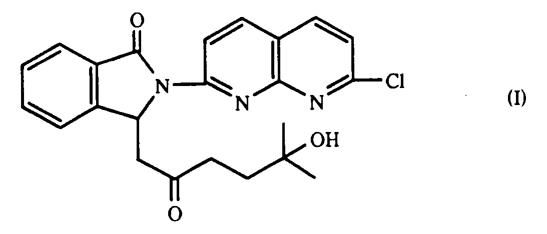
sous forme racémique ou sous forme de ses énantiomères, ainsi que ses sels, sa préparation et les compositions pharmaceutiques qui le contiennent.

Le produit de formule (I) présente des propriétés anxiolytique, hypnotique, anticonvulsivante, antiépileptique et myorelaxante remarquables.

Dans le brevet européen EP 0 274 930 ont été décrits des dérivés de l'isoindolinone de formule générale : 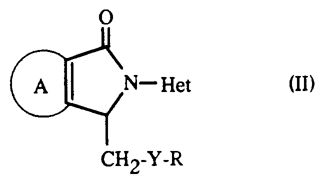
dans laquelle, plus particulièrement, A forme avec le cycle pyrrole un noyau isoindoline, Het représente un radical naphtyridinyle substitué par un atome d'halogène, Y représente un radical CO et R représente un radical alcoyle contenant 1 à 10 atomes de carbone non substitué ou substitué, qui présentent des propriétés thérapeutiques remarquables.

Parmi ces produits, celui pour lequel A forme avec le cycle pyrrole un noyau isoindoline, Het représente un radical (chloro-7 naphtyridine-1,8 yl)-2, Y représente un radical CO et R représente un radical méthyl-3 butyle, sous forme racémique ou sous forme de ses énantiomères, en particulier l'isomère dextrogyre manifeste une activité particulièrement puissante associée à une faible toxicité. En revanche, les produits de formule générale (II) pour lesquels R représente un radical alcoyle substitué par un radical hydroxy primaire tel qu'un radical hydroxy-3 propyle manifestent le même type d'activité à un degré nettement moindre et ils sont de ce fait difficilement exploitables dans l'industrie pharmaceutique.

Il a maintenant été trouvé, et c'est ce qui fait l'objet de la présente invention, que les produits de formule générale (II) dans lesquels R représente un radical alcoyle substitué par un radical hydroxy tertiaire, et en particulier le produit de formule (I) sous forme racémique ou sous forme de ses énantiomères manifeste, de façon surprenante et inattendue, une activité au moins aussi intéressante que celle du produit correspondant pour lequel R représente un radical méthyl-3 butyle.

Selon la présente invention, le produit de formule (I), sous forme racémique, peut être obtenu par hydratation du produit de formule : 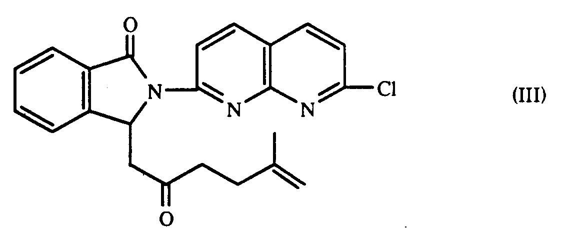
sous forme racémique en opérant en présence d'un acide en milieu hydroorganique.

Comme solvant organique, on utilise de préférence un éther tel que le tétrahydrofuranne ou le dioxanne.

Comme acide, on utilise de préférence un acide minéral tel que l'acide sulfurique.

Généralement, l'hydratation est effectuée à une température comprise entre 0 et 50°C et, de préférence, voisine de 20°C.

Les énantiomères du produit de formule (I) peuvent être obtenus :
- soit par séparation sur phase chirale appropriée des énantiomères constituant le produit de formule (I) racémique,
- soit par séparation sur phase chirale appropriée des énantiomères constituant le produit de formule (III) racémique, suivie de l'hydratation de chacun des énantiomères obtenus.

Généralement, la séparation des énantiomères constituant le produit de formule (I) racémique est effectuée par chromatographie liquide à haute performance sur une colonne de silice enrobée de trisphénylcarbamate de cellulose en éluant avec un solvant convenable tel qu'un mélange éthanol-hexane. De préférence, on utilise une phase préparée dans les conditions décrites dans J. Amer. Chem. Soc., 106, 5357 (1984).

Généralement, la séparation des énantiomères constituant le produit de formule (III) racémique est effectuée par chromatographie liquide à haute performance sur une phase de type Pirkle modifiée en éluant avec un solvant convenable tel qu'un mélange hexane-chlorure de méthylène.

Comme phase chirale, on utilise de préférence une phase dont le sélecteur chiral, qui est, de préférence, la dinitro-3,5 benzoyl-L-leucine, est éloigné de la silice par un bras aminoalcanoyle contenant 3 à 14 atomes de carbone fixé sur les fonctions amines d'une silice aminopropyle et dont les fonctions silanols libres sont bloquées par des radicaux trialcoylsilyles.

Cette phase chirale, qui constitue un autre objet de la présente invention, peut être définie par la structure suivante : 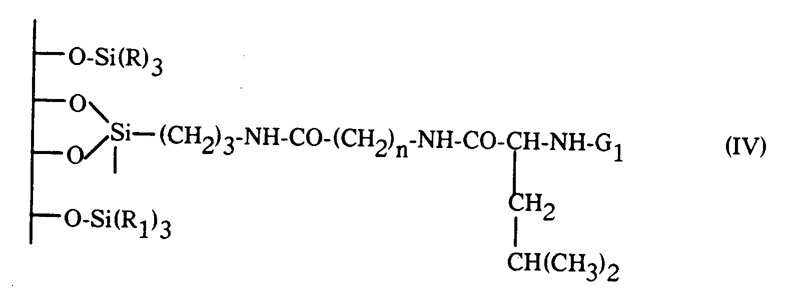
dans laquelle les symboles R, identiques ou différents, et R₁, identiques ou différents, représentent des radicaux alcoyles contenant 1 à 10 atomes de carbone, G₁ représente un groupement électro-attracteur et n représente un nombre entier compris entre 3 et 13 inclusivement.

De préférence, l'un des symboles R représente un radical alcoyle contenant 7 à 10 atomes de carbone et les deux autres représentent un radical alcoyle contenant 1 ou 2 atomes de carbone et de préférence un radical méthyle, les symboles R₁ sont identiques et représentent un radical méthyle ou éthyle, G₁ représente un radical benzoyle éventuellement substitué, de préférence, par un ou plusieurs radicaux nitro tels que le radical dinitro-3,5 benzoyle et n est égal à 10.

La nouvelle phase chirale selon l'invention peut être préparée par action sur une silice aminopropyle de l'anhydride d'un acide aminoalcanoïque contenant 3 à 14 atomes de carbone dont la fonction amine est protégée par un groupement protecteur tel que le radical tert.butoxycarbonyle, suivie du blocage d'une partie des fonctions silanols par des radicaux Si(R)₃ tels que définis précédemment, puis, après élimination du groupement protecteur de la fonction amine, de l'amidification au moyen de L-leucine dont la fonction amine est protégée par un groupement électro-attracteur G₁ tel que défini précédemment, et enfin du blocage des fonctions silanols résiduelles par des radicaux Si(R₁)₃ tels que définis précédemment.

Généralement, l'action de l'anhydride d'un acide aminoalcanoïque protégé sur la silice aminopropyle est effectuée en opérant dans un solvant organique anhydre tel que le diméthylformamide à une température voisine de 20°C.

Le blocage des fonctions silanols par des groupements -Si(R₃) tels que définis précédemment est effectuée par action d'un halogénotrialkylsilane sur la silice aminopropyle greffée par des restes aminoalcanoyles en opérant dans un solvant organique tel que le chlorure de méthylène en présence d'un agent basique tel que la pyridine.

L'élimination des groupements protecteurs des restes aminoalcanoyles s'effectue généralement, lorsque le groupement protecteur est un radical tert.butoxycarbonyle, par action de l'acide trifluoroacétique dans un solvant organique tel que le chlorure de méthylène.

L'amidification au moyen de la L-leucine dont la fonction amine est protégée est effectuée en présence d'un agent de condensation tel que la N-éthoxycarbonyl éthoxy-2 dihydro-1,2 quinoléine en opérant dans un solvant organique anhydre tel que le diméthylformamide.

Le blocage des fonctions silanols résiduelles par des radicaux -Si(R₁)₃ tels que définis précédemment est généralement effectuée au moyen de trialkylsilylimidazole en opérant dans un solvant organique tel que le chlorure de méthylène.

L'hydratation de chacun des énantiomères obtenus est effectuée dans les conditions décrites précédemment pour l'hydratation du produit racémique correspondant.

Le produit de formule (III) peut être obtenu par déalcoxycarbonylation d'un produit de formule générale : 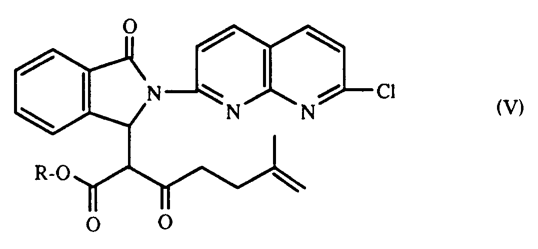
dans laquelle R représente un radical alcoyle droit ou ramifié contenant 1 à 4 atomes de carbone par action du chlorure de lithium dans un solvant organique tel que le diméthylsulfoxyde à une température comprise entre 0 et 50°C.

Le produit de formule générale (V) peut être obtenu par action d'un ester de formule générale : 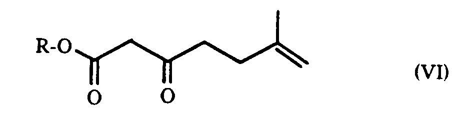
dans laquelle R est défini comme précédemment sur la chloro-3 (chloro-7 naphtyridine-1,8 yl-2)-2 isoindolinone-1 en opérant en présence d'une base minérale forte telle que l'hydrure de sodium dans un solvant organique anhydre tel que le diméthylformamide.

L'ester de formule générale (VI) peut être obtenu dans les conditions décrites par V.B. RAO et coll.,J. Amer. Chem. Soc., 107, 5732 (1985).

La chloro-3 (chloro-7 naphtyridine-1,8 yl-2)-2 isoindolinone-1 peut être préparée dans les conditions décrites dans le brevet européen EP 0 274 930.

Le produit de formule (I), sous forme de racémique et ses énantiomères, en particulier l'énantiomère dextrogyre, éventuellement sous forme de sels, présentent des propriétés pharmacologiques particulièrement intéressantes qui les rendent utiles comme anxiolytique, hypnotique, anti-convulsivant, antiépileptique et myorelaxant. En particulier, ils présentent une bonne affinité in vitro pour les sites récepteurs à benzodiazépines à des concentrations dont les valeurs sont comprises entre 0,5 et 1 nM selon la technique décrite par J.C. Blanchard et L. Julou, J. of Neurochemistry, 40, 601 (1983) inspirée des travaux de Squires et Braestrup, Nature, 266, 732 (1977).

Chez la souris, ils se sont montrés actifs à des doses généralement comprises entre 0,05 et 0,5 mg/kg par voie orale vis-à-vis des convulsions induites par le pentétrazol selon une technique voisine de celle de Everett et Richards, J. Pharmacol., 81, 402 (1944).

Le nouveau produit de formule (I) et ses sels présentent une toxicité faible. Leur DL₅₀ est supérieure à 300 mg/kg par voie orale chez la souris.

Pour l'emploi médicinal, il peut être fait usage du nouveau produit de formule (I) tel quel ou à l'état de sel pharmaceutiquement acceptable, c'est-à-dire non toxique aux doses d'utilisation.

Comme sels pharmaceutiquement acceptables peuvent être cités les sels d'addition avec les acides minéraux tels que chlorhydrates, sulfates, nitrates, phosphates ou les acides organiques tels que les acétates, propionates, succinates, benzoates, fumarates, maléates, méthanesulfonates, iséthionates, théophylline-acétates, salicylates, phénolphtaléinates, méthylène-bis-β-oxynaphtoates ou des dérivés de substitution de ces composés.

Les exemples suivants illustrent la présente invention.

### EXEMPLE 1

A une Suspension de 2,0 g de (chloro-7 naphtyridine-1,8 yl-2)-2 (méthyl-5 oxo-2 hexène-5 yl-1)-3 isoindolinone-1-(R,S) dans 20 cm3 de dioxanne-1,4, on ajoute, à une température voisine de 20°C, 20 cm3 d'une solution aqueuse d'acide sulfurique à 35 % (p/v). La solution jaune obtenue est agitée pendant 12 heures puis on ajoute 20 g de glace pilée. Le pH du mélange réactionnel est amené au voisinage de 7 par addition d'une solution aqueuse de soude 1N. On extrait alors par 3 fois 100 cm3 d'acétate d'éthyle. Les phases organiques réunies sont lavées par 2 fois 50 cm3 d'eau, séchées sur sulfate de magnésium et concentrées à sec sous pression réduite à une température voisine de 40°C. Après recristallisation du résidu obtenu dans l'acétonitrile, on obtient 1,2 g de (chlor-7 naphtyridine-1,8 yl-2)-2 (méthyl-5 hydroxy-5 oxo-2 hexyl)-3 isoindolinone-1-(R,S) fondant à 140°C.

La (chloro-7 naphtyridine-1,8 yl-2)-2 (méthyl-5 oxo-2 hexène-5 yl-1)-3 isoindolinone-1-(R,S) peut être préparée de la manière suivante :
A une solution de 9,3 g de [(chloro-7 naphtyridine-1,8 yl-2)-2 oxo-3 isoindolinyl-1]-2 méthyl-6 oxo-3 heptène-6 oate d'éthyle (mélange des formes A et B) dans 60 cm3 de diméthylsulfoxyde, on ajoute à une température voisine de 25°C, 11,5 g de chlorure de lithium et 4,6 cm3 d'eau. Le mélange réactionnel est chauffé à reflux pendant 20 minutes, refroidi à une température voisine de 30°C, dilué par 60 cm3 d'eau puis refroidi à une température voisine de 5°C. Le solide précipité est séparé par filtration, lavé successivement par 2 fois 25 cm3 d'eau, 1 fois 20 cm3 d'éthanol et 2 fois 25 cm3 d'oxyde de diisopropyle. On obtient ainsi, après séchage à l'air, 7,2 g de (chloro-7 naphtyridine-1,8 yl-2)-2 (méthyl-5 oxo-2 hexène-5 yl-1)-3 isoindolinone-1-(R,S) fondant à 184°C.

Le [(chloro-7 naphtyridine-1,8 yl-2)-2 oxo-3 isoindolinyl-1]-2 méthyl-6 oxo-3 heptène-6 oate d'éthyle (mélange des formes A et B) peut être préparé de la manière suivante :
On ajoute 1,7 g de suspension huileuse (50 % en poids) d'hydrure de sodium à 100 cm3 de diméthylformamide anhydre, sous atmosphère d'argon, à une température voisine de 10°C. On ajoute ensuite 9,8 g de méthyl-6 oxo-3 heptène-6 oate d'éthyle dans 25 cm3 de diméthylformamide anhydre. La suspension obtenue est agitée pendant 30 minutes en laissant remonter la température vers 20°C puis on ajoute 11,6 g de chloro-3 (chloro-7 naphtyridine-1,8 yl-2)-2 isoindolinone-1. Le mélange réactionnel est agité pendant 4 heures à une température voisine de 20°C puis versé dans 250 cm3 d'eau. La phase aqueuse, acidifiée par addition de 25 cm3 d'une solution aqueuse d'acide chlorhydrique 1N, est extraite par 2 fois 200 cm3 de dichlorométhane. Les phases organiques réunies sont lavées par 2 fois 50 cm3 d'eau, séchées sur sulfate de magnésium et concentrées à sec sous pression réduite à 40°C. Le résidu huileux obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi, après cristallisation dans l'oxyde de diisopropyle, 9,4 g de [(chloro-7 naphtyridine-1,8 yl-2)-2 oxo-3 isoindolinyl-1]-2 méthyl-6 oxo-3 heptène-6 oate d'éthyle (mélange des formes A et B) fondant à 125°C.

Le méthyl-6 oxo-3 heptène-6 oate d'éthyle peut être préparé selon la méthode décrite par V.B. RAO et coll., J. Amer. Chem. Soc., 107, 5732 (1985).

La chloro-3 (chloro-7 naphtyridine-1,8 yl-2)-2 isoindolinone-1 peut être préparée selon la méthode décrite dans le brevet européen EP 0 274 930.

### EXEMPLE 2

En partant de 0,86 g de (chloro-7 naphtyridine-1,8 yl-2)-2 (méthyl-5 hydroxy-5 oxo-2 hexyl)-3 isoindolinone-1-(R,S), on sépare par chromatographie liquide à haute performance sur 350 g de support constitué de silice enrobée de trisphénylcarbamate de cellulose préparé selon J. Amer. Chem. Soc., 106, 5357 (1984) et contenu dans une colonne de 19 cm de longueur et de 6 cm de diamètre avec comme phase mobile le mélange éthanol-hexane (1-1 en volumes) au débit de 30 cm3/minute en éluant successivement :
- 0,38 g de (-)-(chloro-7 naphtyridine-1,8 yl-2)-2 (méthyl-5 hydroxy-5 oxo-2 hexyl)-3 isoindolinone-1 fondant à 178°C dont le pouvoir rotatoire est [α]²⁰_{D} = -105° ± 2° (c = 0,75 ; chloroforme), puis
- 0,41 g de (+)-(chloro-7 naphtyridine-1,8 yl-2)-2 (méthyl-5 hydroxy- oxo-2 hexyl)-3 isoindolinone-1 fondant à 178°C dont le pouvoir rotatoire est [α]²⁰_{D} = +104° ± 2° (c = 0,81 ; chloroforme)

### EXEMPLE 3

En opérant comme dans l'exemple 1, mais à partir de 40 g de (+)-(chloro-7 naphtyridine-1,8 yl-2)-2 (méthyl-5 oxo-2 hexène-5 yl-1)-3 isoindolinone-1, on obtient 8 mg de (+)-(chloro-7 naphtyridine-1,8 yl-2)-2 (méthyl-5 hydroxy-5 oxo-2 hexyl)-3 isoindolinone-1 dont les caractéristiques sont identiques à celle du produit obtenu à l'exemple 2.

La (+)-(chloro-7 naphtyridine-1,8 yl-2)-2 (méthyl-5 oxo-2 hexène-5 yl-1)-3 isoindolinone-1 peut être obtenue de la manière suivante :
En partant de 2,8 g de (chloro-7 naphryridine-1,8 yl-2)-2 (méthyl-5 oxo-2 hexène-5 yl-1)-3 isoindolinone-1-(R,S), on sépare par chromatographie liquide à haute performance sur 400 g de support, dont la préparation est décrite ci-après, contenu dans une colonne de 26 cm de longueur et de 6 cm de diamètre avec comme phase mobile le mélange hexane-chlorure de méthylène (100-15 en volumes) au débit de 60 cm3/minute en éluant successivement :
- 0,76 g de (+)-(chloro-7 naphtyridine-1,8 yl-2)-2 (méthyl-5 oxo-2 hexène-5 yl-1)-3 isoindolinone-1 fondant à 178°C après recristallisation dans l'éthanol et dont le pouvoir rotatoire est [α]²⁰_{D} = +141° ± 3° (c = 0,53 ; chloroforme)
- 0,45 g de (-)-(chloro-7 naphtyridine-1,8 yl-2)-2 (méthyl-5 oxo-2 hexène-5 yl-1)-3 isoindolinone-1 fondant à 178°C après recristallisation dans l'éthanol et dont le pouvoir rotatoire est [α]²⁰_{D} = -136° ± 3° (c = 0,43 ; chloroforme).

Le support peut être préparé de la manière suivante :
Dans un tricol de 2 litres, on met en suspension 253 g de silice aminopropyle (100 Å-10 µm-NH₂ ; Macherey-Nagel) dans 850 cm3 de diméthylformamide séché sur tamis moléculaire 4Å. On ajoute 40 g d'anhydride de l'acide N-tert.butoxycarbonyl amino-11 undécanoïque. Le mélange réactionnel est agité pendant 16 heures à 20°C puis est filtré sur verte fritté. La silice est lavée successivement par 150 cm3 de diméthylformamide, 2 fois 200 cm3 de tétrahydrofuranne et 2 fois 200 cm3 de chlorure de méthylène. La silice ainsi lavée est remise en suspension dans 850 cm3 de diméthylformamide. On ajoute 40 g d'anhydride de l'acide N-tert.butoxycarbonyl amino-11 undécanoïque puis on agite le mélange pendant 6 heures à 20°C. On filtre sur verte fritté et lave la silice successivement par 150 cm3 de diméthylformamide, 2 fois 200 cm3 de chlorure de méthylène, 200 cm3 de méthanol et 150 cm3 de chlorure de méthylène. Après séchage de la silice à 20°C sous pression réduite (20 mm de mercure : 2,7 kPa), on obtient 267 g de silice désignée par l'appellation "BOC-C₁₁-C₃- (silice)" sous forme de poudre blanche dont la structure est confirmée par le spectre infra-rouge et dont l'analyse élémentaire (trouvée) est : C % = 8,1 ; H % = 1,7 ; N % = 1,4.

Dans un tricol de 2 litres, on introduit 266 g de silice "BOC-C₁₁-C₃-(silice)", 800 cm3 de chlorure de méthylène et 30 cm3 de pyridine. On ajoute, goutte à goutte, en 10 minutes, 230 cm3 de diméthyloetylchlorosilane puis agite pendant une nuit. Le solide obtenu est séparé par filtration puis lavé successivement par 2 fois 500 cm3 de chlorure de méthylène, 2 fois 500 cm3 de méthanol, 2 fois 500 cm3 de chlorure de méthylène et 2 fois 250 cm3 d'éther éthylique. Après séchage à 20°C sous pression réduite (20 mm de mercure ; 2,7 kPa), on obtient 274 g de silice désignée par l'appellation "BOC-C₁₁-C₃- (silice)-O-Si(CH₃)₂(CH₂)₇CH₃" dont la structure est confirmée par le spectre infra-rouge et dont l'analyse élémentaire (trouvée) est : C % = 10,0 ; H % 2,1 ; N % = 1,3.

Dans un tricol de 2 litres, on met en suspension 274 g de silice "BOC-C₁₁-C₃- (silice)-O-Si(CH₃)₂(CH₂)₇CH₃" dans 800 cm3 d'une solution à 6 % en volumes d'acide trifluoroacétique dans le chlorure de méthylène. On agite le mélange réactionnel pendant 20 heures à 20°C. La silice est séparée par filtration sur verte fritté, lavée par 2 fois 300 cm3 de chlorure de méthylène, 2 fois 500 cm3 d'une solution à 30 % de diisopropyléthylamine dans le chlorure de méthylène pendant 30 minutes, 2 fois 500 cm3 de méthanol et 500 cm3 d'éther éthylique. Après séchage à 20°C sous pression réduite (20 mm de mercure ; 2,7 kPa), on obtient 269 g de silice désignée par l'appellation "C₁₁-C₃- (silice)-O-Si(CH₃)₂(CH₂)₇CH₃" dont l'analyse élémentaire (trouvée) est : C % = 8,4 ; H % = 1,7 ; N % = 1,2.

Dans un ballon tricol de 2 litres, on met en suspension 266 g de silice "C₁₁-C₃- (silice)-O-Si(CH₃)₂(CH₂)₇CH₃" dans 800 cm3 de diméthylformamide séché sur tamis moléculaire 4Å. On ajoute 27,5 g de dinitro-3,5 benzoyl-L-leucine et 20 g de N-éthoxycarbonyl éthoxy-2 dihydro-1,2 quinoléine. On agite le mélange réactionnel pendant 1 nuit. La silice est séparée par filtration sur verte fritté puis est lavée par 2 fois 500 cm3 de chlorure de méthylène, 2 fois 500 cm3 de tétrahydrofuranne, 500 cm3 de diméthylformamide et 500 cm3 de chlorure de méthylène. La silice ainsi lavée est remise en suspension dans 800 cm3 de diméthylformamide. On ajoute successivement 27,5 g de dinitro-3,5 benzoyl-L-leucine et 20 g de N-éthoxycarbonyl éthoxy-2 dihydro-1,2 quinoléine puis on agite pendant une nuit à 20°C. La silice est séparée par filtration sur verte fritté et est lavée successivement par 2 fois 500 cm3 de chlorure de méthylène, 2 fois 500 cm3 de tétrahydrofuranne, 2 fois 500 cm3 de méthanol et 2 fois 500 cm3 d'éther éthylique. Après séchage à 60°C sous pression réduite (20 mm de mercure; 2,7 kPa), on obtient 273 g de silice désignée par l'appellation "DBN-L-Leu-C₁₁-C₃ (silice)-O-Si(CH₃)₂(CH₂)₇CH₃" que l'on remet en suspension dans 800 cm3 de chlorure de méthylène. On ajoute 350 cm3 de triméthylsilylimidazole en 15 minutes puis on agite pendant 1 nuit. La silice est séparée par filtration et est lavée successivement par 2 fois 300 cm3 de tétrahydrofuranne, 2 fois 300 cm3 de méthanol, 2 fois 300 cm3 d'acétone et 2 fois 300 cm3 de chlorure de méthylène. Après séchage à 60°C sous pression réduite (20 mm de mercure ; 2,7 kPa), on obtient 275 g de silice désignée par l'appellation "DBN-L-Leu-C₁₁-C₃- (silice)-[O-Si-(CH₃)₂(CH₂)₇CH₃]-[O-Si(CH₃)₃]" sous forme d'une poudre jaune pâle dont la structure est confirmée par le spectre infra-rouge et dont l'analyse élémentaire (trouvée) est : C % = 12,8 ; H % = 2,3 ; N % = 2,2.

L'anhydride de l'acide N-tert.butoxycarbonyl amino-11 undécanoïque peut être préparé de la manière suivante :
On dissout 30,1 g d'acide N-tert.butoxycarbonyl amino-11 undécanoïque dans 480 cm3 d'acétate d'éthyle. On refroidit cette solution à 5°C puis, en la maintenant à cette température, on ajoute en 10 minutes une solution de 10,63 g de dicyclohexylcarbodiimide dans 120 cm3 d'acétate d'éthyle. On agite le mélange réactionnel pendant 1 heure à 5°C puis pendant 16 heures à une température voisine de 20°C. On sépare le précipité formé par filtration et le lave par 30 cm3 d'acétate d'éthyle. Le filtrat est concentré à sec sous pression réduite (20 mm de mercure ; 2,7 kPa) à 30°C. Le solide obtenu est séché à 20°C sous pression réduite (20 mm de mercure ; 2,7 kPa). On obtient ainsi, avec un rendement voisin de 100 %, 31 g d'anhydride de l'acide N-tert.butoxycarbonyl amino-11 undécanoïque.

L'acide N-tert.butoxycarbonyl amino-11 undécanoïque peut être préparé de la manière suivante :
Dans un tricol de 4 litres muni d'une agitation mécanique et d'un réfrigérant, on introduit successivement 160 g d'acide amino-11 undécanoïque, 2 litres de dioxanne, 1,3 litre d'eau distillée, 208 g de carbonate de sodium et 173 g de di-tert.butyl dicarbonate. Le mélange réactionnel est chauffé à l'ébullition pendant 16 heures. On obtient ainsi une solution limpide. Après refroidissement à 20°C, le mélange réactionnel est versé sur 800 g de glace puis acidifié à pH = 3-4 par addition d'acide chlorhydrique 4N. On obtient ainsi un précipité blanc qui est séparé par filtration, lavé par 300 cm3 d'eau et séché à 20°C sous pression réduite (20 mm de mercure ; 2,7 kPa). On obtient ainsi, avec un rendement de 95 %, 232 g d'acide N-tert.butoxycarbonyl amino-11 undécanoïque dont le point de fusion (68°C) est en accord avec celui qui est donné dans J. Org. Chem., 41, 1350 (1976).

La présente invention concerne également les médicaments qui contiennent les produits de formule (I) à l'état pur ou sous forme de compositions dans lesquelles ils sont associés à un adjuvant, un diluant et/ou un enrobage compatibles et pharmaceutiquement acceptables. Ces médicaments peuvent être employés par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine pour leur action anxiolytique, hypnotique, anticonvulsivante, antiépileptique et myorelaxante.

En thérapeutique humaine, les doses dépendent de l'effet recherché et de la durée du traitement ; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### EXEMPLE

On prépare selon la technique habituelle des comprimés dosés à 10 mg de produit actif ayant la composition suivante :

| | |
|---|---|
| - (chloro-7 naphtyridine-1,8 yl-2)-2 (méthyl-5 hydroxy-5 oxo-2 hexyl)-3 isoindolinone-1 | 0,01 g |
| - amidon | 0,200 g |
| - silice précipitée | 0,036 g |
| - stéarate de magnésium | 0,004 g |

## Revendications

1. Dérivé de l'isoindolinone de formule : 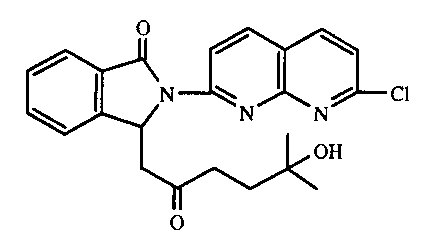 sous forme racémique ou sous forme de ses énantiomères, ainsi que ses sels.

2. Procédé de préparation du produit selon la revendication 1 sous forme racémique caractérisé en ce que l'on effectue l'hydratation d'un produit de formule : 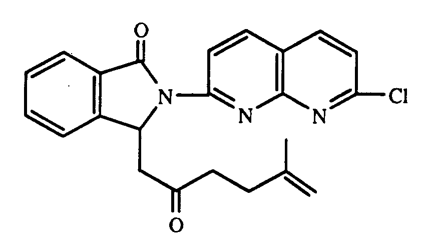 sous forme racémique, en présence d'un acide en milieu hydroorganique.

3. Procédé de préparation des énantiomères du produit selon la revendication 1 caractérisé en ce que l'on effectue la séparation des constituants du produit selon la revendication 1 racémique par chromatographie liquide à haute performance sur une colonne de silice enrobée de trisphénylcarbamate de cellulose.

4. Procédé de préparation des énantiomères du produit selon la revendication 1 caractérisé en ce que l'on sépare des constituants du produit racémique de formule : 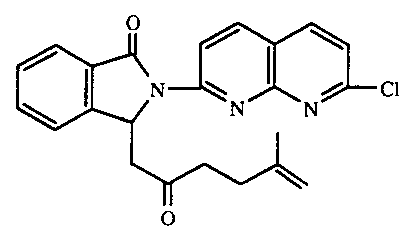 par chromatographie liquide à haute performance sur une phase de type Pirkle modifiée, puis hydrate chacun des énantiomères ainsi obtenus.

5. Procédé selon la revendication 4 caractérisé en ce que la phase de type Pirkle modifiée est définie par la structure : 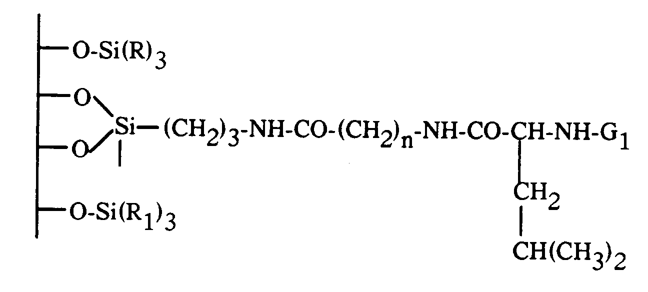 dans laquelle les symboles R, identiques ou différents, et R₁, identiques ou différents, représentent des radicaux alcoyles contenant 1 à 10 atomes de carbone, G₁ représente un groupement électro-attracteur et n représente un nombre entier compris entre 3 et 13 inclusivement.

6. Procédé selon la revendication 4 caractérisé en ce que la phase de type Pirkle modifiée est définie par la structure : 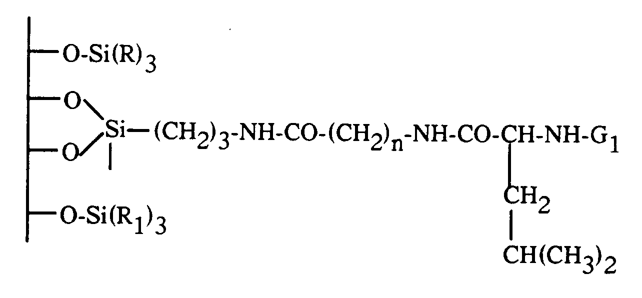 dans laquelle l'un des symboles R représente un radical alcoyle contenant 7 à 10 atomes de carbone et les deux autres représentent un radical alcoyle contenant 1 ou 2 atomes de carbone, Tes symboles R₁ sont identiques et représentent un radical méthyle ou éthyle, G₁ représente un radical benzoyle éventuellement substitué par un ou plusieurs radicaux nitro et n est égal à 10.

7. Procédé selon la revendication 4 caractérisé en ce que la phase Pirkle modifiée est définie par la structure :

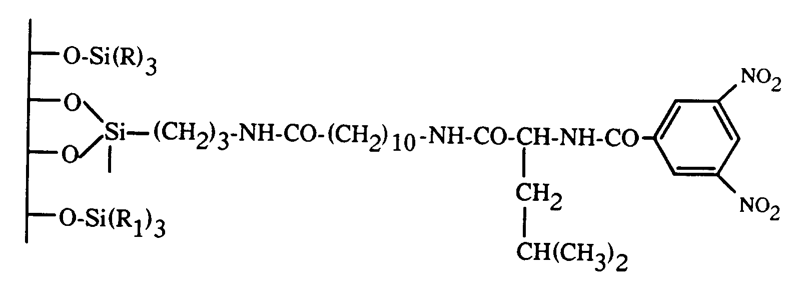

8. La phase de type Pirkle modifiée telle qu'elle est définie selon l'une des revendications 5, 6 ou 7.

9. Composition pharmaceutique caractérisée en ce qu'elle contient au moins un produit selon la revendication 1 sous forme racémique ou sous forme de ses énantiomères en association avec un ou plusieurs diluants ou adjuvants pharmaceutiquement acceptables inertes ou thérapeutiquement actifs.

## Claims

1. Isoindolinone derivative of formula: 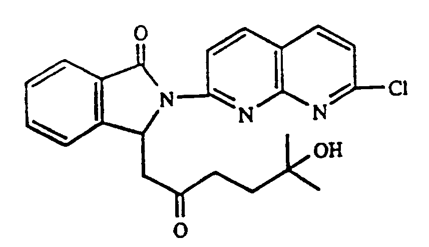 in racemic form or in the form of its enantiomers, as well as its salts.

2. Process for preparing the product according to Claim 1 in racemic form, characterized in that the hydration of a product of formula: 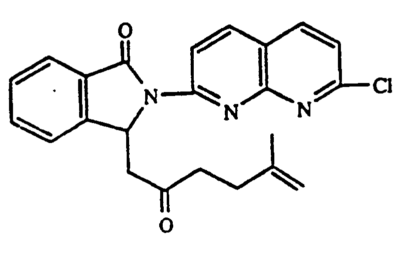 in racemic form, is carried out in the presence of an acid in an aqueous-organic medium.

3. Process for preparing the enantiomers of the product according to Claim 1, characterized in that the separation of the constituents of the racemic product according to Claim 1 is carried out by high-performance liquid chromatography on a silica column coated with cellulose triphenylcarbamate.

4. Process for preparing the enantiomers of the product according to Claim 1, characterized in that the constituents of the racemic product of formula: 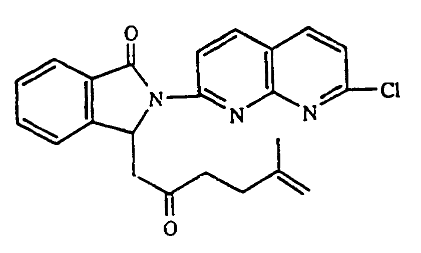 are separated by high-performance liquid chromatography on a modified Pirkle-type phase and then each of the enantiomers thus obtained is hydrated.

5. Process according to Claim 4, characterized in that the modified Pirkle type phase is defined by the structure: 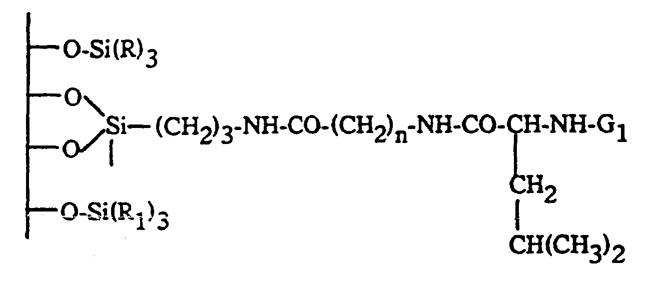 in which the symbols R, which are identical or different, and R₁, which are identical or different, represent alkyl radicals containing 1 to 10 carbon atoms, G₁ represents an electron-attracting group and n represents an integer between 3 and 13 inclusive.

6. Process according to Claim 4, characterized in that the modified Pirkle-type phase is defined by the structure: 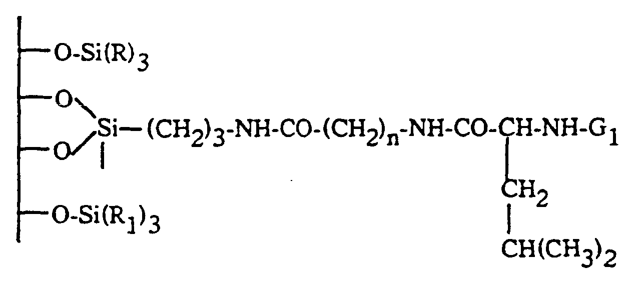 in which one of the symbols R represents an alkyl radical containing 7 to 10 carbon atoms and the other two represent an alkyl radical containing 1 or 2 carbon atoms, the symbols R₁ are identical and represent a methyl or ethyl radical, G₁ represents a benzoyl radical optionally substituted by one or more nitro radicals and n is equal to 10.

7. Process according to Claim 4, characterized in that the modified Pirkle phase is defined by the structure:

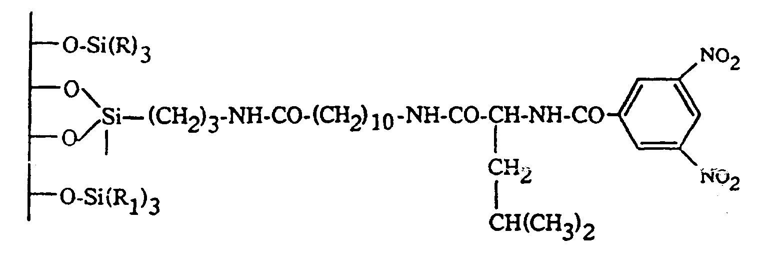

8. The modified Pirkle-type phase as defined according to one of Claims 5, 6 or 7.

9. Pharmaceutical composition characterized in that it contains at least one product according to Claim 1 in racemic form or in the form of its enantiomers in combination with one or more pharmaceutically acceptable inert or therapeutically active diluents or adjuvants.

## Patentansprüche

1. Derivat des Isoindolinons der Formel 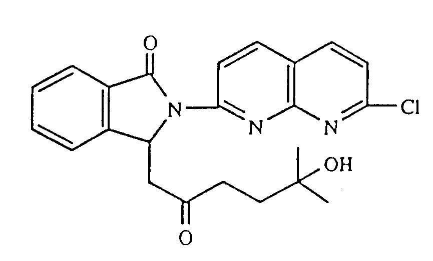 in razemischer Form oder in Form seiner Enantiomeren sowie seiner Salze.

2. Verfahren zur Herstellung des Produkts gemäß Anspruch 1 in razemischer Form, dadurch gekennzeichnet, daß man die Hydratation eines Produkts der Formel 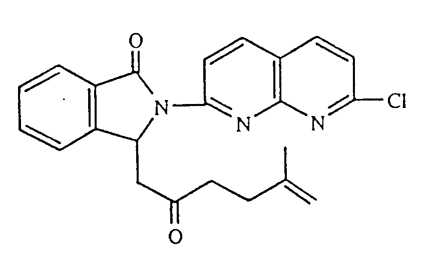 in razemischer Form in Gegenwart einer Säure in hydroorganischem Milieu bewirkt.

3. Verfahren zur Herstellung der Enantiomeren des Produkts gemäß Anspruch 1, dadurch gekennzeichnet, daß man die Trennung der Bestandteile des razemischen Produkts gemäß Anspruch 1 durch Hochleistungsflüssigkeitschromatographie auf einer Säule von Siliziumdioxyd, das mit Zellulosetrisphenylcarbamat umhüllt ist, vornimmt.

4. Verfahren zur Herstellung der Enantiomeren des Produkts gemäß Anspruch 1, dadurch gekennzeichnet, daß man Bestandteile des razemischen Produkts der Formel 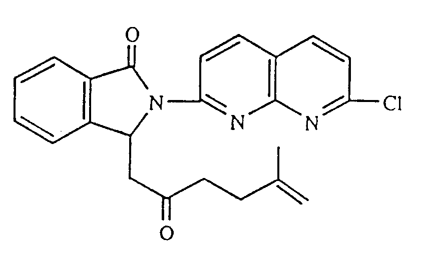 durch Hochleistungsflüssigkeitschromatographie auf einer modifizierten Phase vom Typ Pirkle trennt und dann jedes der so erhaltenen Enantiomeren hydratisiert.

5. Verfahren gemäß Anspruch 4, dadurch gekennzeichnet, daß die modifizierte Phase vom Typ Pirkle durch die Struktur 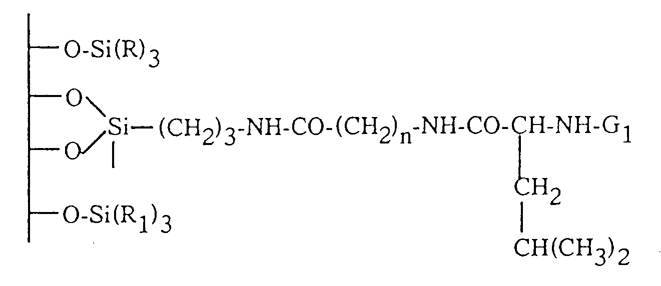 definiert ist, worin die Symbole R, die identisch oder verschieden sind, und R₁, die identisch oder verschieden sind, Alkylreste mit 1-10 Kohlenstoffatomen bedeuten, G₁ bedeutet eine elektroanziehende Gruppe, und n bedeutet eine ganze Zahl zwischen 3 und 13 einschließlich.

6. Verfahren gemäß Anspruch 4, dadurch gekennzeichnet, daß die modifizierte Phase vom Typ Pirkle durch die Struktur 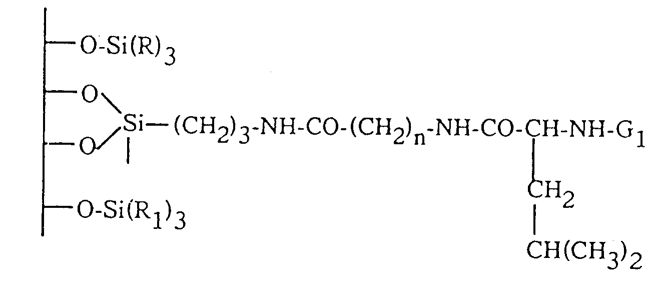 definiert ist, worin eines der Symbole R einen Alkylrest mit 7-10 Kohlenstoffatomen bedeutet und die beiden anderen einen Alkylrest mit 1 oder 2 Kohlenstoffatomen bedeuten, die Symbole R₁ identisch sind und einen Methyl- oder Ethylrest bedeuten, G₁ bedeutet einen gegebenenfalls durch ein oder mehrere Nitroreste substituierten Benzoylrest, und n ist gleich 10.

7. Verfahren gemäß Anspruch 4, dadurch gekennzeichnet, daß die modifizierte Pirklephase definiert ist durch die Struktur:

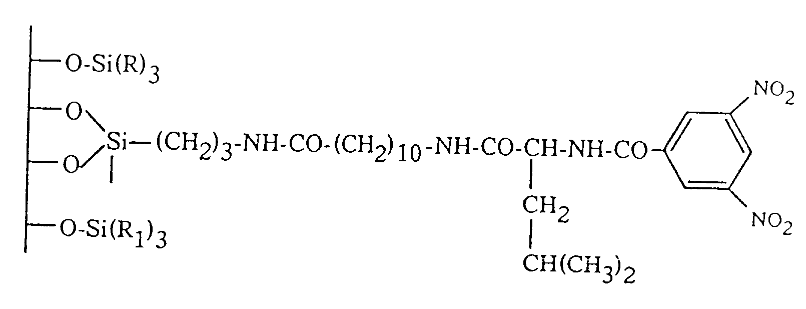

8. Die modifizierte Phase vom Typ Pirkle, wie sie gemäß einem der Ansprüche 5, 6 oder 7 definiert ist.

9. Pharmazeutische Zusammensetzung, dadurch gekennzeichnet, daß sie mindestens ein Produkt gemäß Anspruch 1 in razemischer Form oder in Form seiner Enantiomeren in Assoziation mit einem oder mehreren, pharmazeutisch annehmbaren inerten oder therapeutisch aktiven Verdünnungs- oder Hilfsmitteln enthält.
